# EUROPEAN PATENT APPLICATION

(11) **EP 4 817 641 A1**
(43) Date of publication of application: **07.10.2026**
(21) Application number: 26167878.3
(22) Date of filing: 26.03.2026
(51) Int. Cl.: A61L 15/26

(54) **MEDICAL DEVICE FOR SKIN APPLICATION AND RELATED MANUFACTURING PROCEDURE**

(30) Priority: 04.04.2025 IT 202500007380
(71) Applicant: A.P.R. Meds Srl, 26900 Lodi (LO) (IT)
(72) Inventor: SERIO, Francesco, 26900 LODI (LO) (IT); CARIONI, Gianluigi, 26900 LODI (LO) (IT); D'ERRICO, Fabrizio, 26900 LODI (LO) (IT)
(74) Representative: Mele, Emanuele

(57) **Abstract**

The present invention relates to a medical device for cutaneous application. The present invention also relates to a process for the realization of such a device.

## Description

### Field of the invention

The present invention relates to a medical device for cutaneous application. The present invention also relates to a process for the realization of such a device.

### Background

As is known, the application of microcurrents in the therapeutic and rehabilitative field is widely documented in the scientific and clinical literature for its ability to interact with the extracellular ionic microenvironment and therefore modulate the activity of cellular membranes. In particular, the application of microcurrents at the cutaneous or subcutaneous level has shown relevant effects on the neuromodulative and reparative level, both in nervous and connective tissues. Consolidated studies demonstrate that the introduction of external electrical signals, even at low intensity, is able to activate physiological responses related to ionic rebalancing, membrane stabilization and guided cellular migration [1][2].

A recent systematic review has confirmed that, despite the heterogeneity of the protocols, the effect of microcurrents appears promising in the stimulation of the neuro-muscular response and in the modulation of the extracellular environment [3].

The biophysical mechanisms involved, according to consolidated theories also in the cerebral neuromodulative field, are based on the controlled alteration of membrane potentials, on the local ionotropic action and on the possible electrochemical transduction in depth, even in the absence of active generation of the action potential [4].

Physical therapies that employ microcurrents - typically in the intensity range between 10 and 500 µA - act predominantly at the level of the extracellular matrix of soft tissues, of the superficial and deep connective fascia, and finally of the cellular membranes of excitable cells (neurons, myocytes), where they determine a redistribution of ionic charges (especially Na⁺, K⁺ and Ca²⁺) and favor the restoration of the physiological membrane potential.

Such effects are made possible by the fact that the connective tissue - and in particular the extracellular matrix that directly surrounds the nerve endings and the peripheral neuronal structures - constitutes an electrochemical and functional continuum with the neuronal membrane. This continuity is at the basis of the local homeostatic regulation and allows the modulation of the bio-electric field - deriving from the ionic concentration in the extracellular environment - which in turn is reflected directly by remodulating the tissue membrane potential towards the condition of resting membrane potential. In the presence of perturbations (inflammatory, traumatic, neuro-degenerative), the breaking of this equilibrium, in fact, leads to states of chronic depolarization, hyperexcitability or, in severe cases, to the blockage of the transmission of bioelectric signals, all neurophysiological phenomena that micro-currents aim to counteract through the principle of action based on the introduction from the outside of an electric field. The electric field introduced into the tissue determines the microcirculation of bio-currents (that is, based on the ionic flow inside the extracellular fluid) bringing, as a benefit, a redistribution towards the equilibrium of ionic charges present in the same extracellular fluid.

At a functional level, it is known, as well as scientifically and clinically demonstrated, that the ionic micro-currents induced by the two electrodes (anode and cathode) applied to the skin - favoring the restoration of the conditions of ionic equilibrium - are able: a) on non-excitable cells, to increase the mitochondrial synthesis of ATP, repolarize partially depolarized membranes, stimulate the processes of repair and regeneration of tissues, reduce chronic inflammatory states; b) on neuronal excitable cells, they are able to modulate neuronal excitability and therefore muscular.

However, the production of such beneficial effects requires the use of active energy sources, generally based on electronic generators, conductive electrodes, which prevent their autonomy, discouraging ease of use and integration in unassisted clinical and home contexts.

There is therefore the necessity to arrange for an alternative solution that allows obtaining therapeutic effects entirely analogous to those induced by microcurrents, in particular - but not exclusively - as regards the ionic rebalancing of the extracellular matrix (ECM, extracellular matrix).

The extracellular matrix (ECM), which directly surrounds the neuronal bundles and other excitable tissues, represents in general the first level of biophysical interface through which it is possible to actively modulate the membrane potentials of the cells [5][7].

The ECM acts in fact as a mechanical and electrochemical mediator between the external environment and the cellular membrane, thanks to the presence of mechanosensitive ion channels, integrins and signal transduction systems capable of influencing membrane polarization [5][6].

Such mechanisms are involved both in the maintenance of ionic homeostasis in physiological conditions, and in the local electrochemical alterations associated with pathological situations such as inflammation, fibrosis or nerve degeneration [6][7].

To this end, a passive device is necessary, devoid of electronic components or external power supply, capable of acting locally on the skin through a mechanism of induced electric charge. This charge is actually generated by the spontaneous polarization of the material of a dielectric nature constituting the device itself, once placed in direct contact with the human body, that is with the endogenous bioelectric field.

Such an approach allows reproducing the rebalancing effect of microcurrents in a continuous, safe and non-invasive way, significantly improving the usability of the device and its integration in therapeutic contexts, including prolonged and home-based ones.

National patent IT n. 102022000011933 describes a first passive medical device for cutaneous application, based on a principle of capacitive interaction with endogenous bioelectric fields, obtained through a functional layer composed of dielectric material capable of polarizing spontaneously in contact with the skin. Such a device has demonstrated efficacy in modulating the underlying bioelectric activity, in particular in the neuromuscular field.

There is therefore the necessity for an improved medical device compared to the devices of the prior art.

### Summary

The Applicant has now found a new improved medical device. Such a device being able to interact passively with the extracellular ionic microenvironment of biological tissues, without generating exogenous current or using electronic circuits, guaranteeing a non-invasive, continuous, safe treatment and applicable in clinical or home contexts. Furthermore, the device of the invention presents advantageous and marked characteristics of water repellency, resistance to wear from adhesives, as well as high anti-sweat and ergonomic properties.

Therefore, in a first aspect, the present invention relates to a medical device according to claim 1.

The present invention therefore relates to a medical device for cutaneous application comprising a composite material consisting of a cross-linked silicone matrix comprising a stable dispersion of micronized polyester polymer particles incorporated in said matrix, the aforementioned particles having a relative dielectric constant between 2.0 and 5.0 measured at 1 MHz.

Furthermore, in a second object, the present invention relates to a process according to claim 9.

Therefore, the present invention also relates to a process for the realization of the device, said medical device comprising the steps of:
a) fragmentation by mechanical cutting of a layer or multilayer of polyester polymer material having a relative dielectric constant between 2.0 and 5.0 measured at 1 MHz to obtain fragments of the layer or multilayer of polymer material;
b) cryogenic micronization by grinding the fragments referred to in step a) in liquid nitrogen, under conditions below the glass transition point of the polyester polymer material, to obtain micronized particles of linear dimension greater than 1 µm and less than or equal to 20 µm;
c) dispersion of the micronized particles referred to in point b) in a pre-crosslinkable silicone composition containing vinyl groups, in inert conditions and controlled stirring;
d) addition to the composition of a suitable cross-linking agent to trigger the formation of a cross-linked silicone matrix, inside which the micronized particles are permanently incorporated.

Advantageously, the medical device according to the invention is for cutaneous application, capable of interacting passively with the extracellular ionic microenvironment of biological tissues, in particular at the level of the extracellular matrix (ECM) that surrounds the neuronal and muscular structures and the cutaneous receptors (Pacini, Meissner, Ruffini, Merkel), which contain voltage-dependent ion channels present in the free nerve endings, contributing to modulate locally the ionic balance and the membrane potential.

Such a device introduces a new architecture of the device, wherein micronized particles are dispersed within a cross-linked silicone matrix.

This configuration allows maintaining the adaptive dielectric behavior to the tissue bioelectric field, with a stable, controlled polarization response, achieving greater efficiency in the distribution of the self-induced electric field on the cutaneous surface.

Furthermore, while the invention object of the national patent application IT n. 102022000011933 is based on a homogeneous mono-material structure, the current invention realizes an engineered composite system that allows the optimization of the ratio between mechanical flexibility, continuity of polarization, and distribution of charges on the surface in contact with the biological tissues.

Such a configuration, which is not suggested in the previous national patent IT n. 102022000011933, therefore represents an improved solution, which extends the field of application and significantly improves the biophysical performance of the device, without introducing active elements or external electrical power supply.

### Brief description of the figures

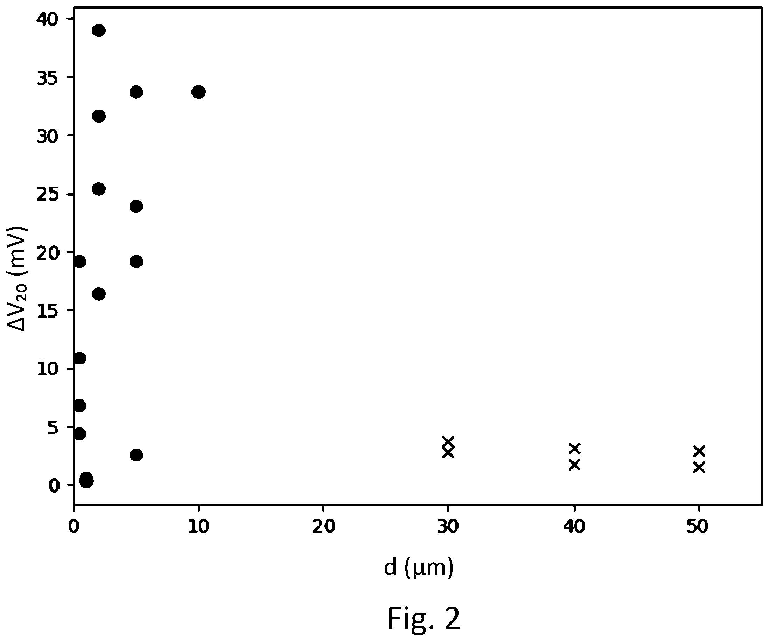

The present invention will now be described for illustrative but not limiting purposes, according to its preferred embodiments, with particular reference to the figure of the attached drawing, wherein:

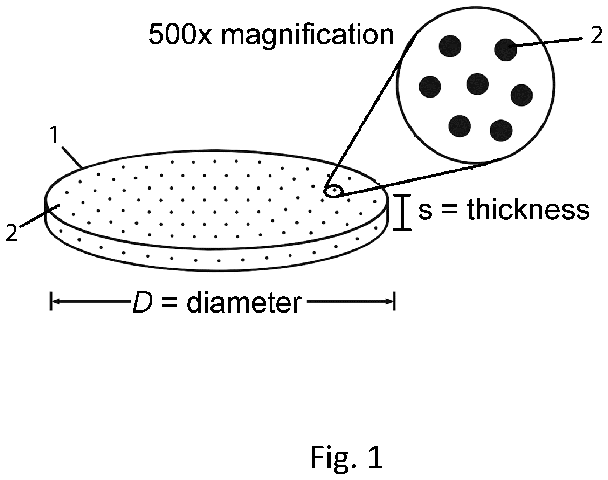

Figure 1 represents an exemplary schematic view of a device according to the invention, in particular in the form of a discoid insert (1) to be applied on the skin. The device consists of a flexible composite containing dispersed particles (2) inside a cross-linked silicone matrix. The schematic magnification (500×) highlights the uniform distribution of the particles (2) inside the volume of the device. Furthermore, the main geometric dimensions of the device are indicated:
D: overall diameter; s: thickness of the composite. Figure 2 is a graph illustrating the non-linear dependence of the potential difference at 20 minutes (ΔV₂₀) on the dimension of the particles (d) used in the device of Figure 1. From the graph, an almost complete collapse of the response is observed for particles with a dimension of about 1 µm, an increase in the response for particles with a dimension ≥ 2 µm, with maximum efficacy in the range 5-10 µm. From the graph, a second collapse of the response is also observed for particles of larger dimensions (30-50 µm), where the measured voltage is reduced to values comparable to those of the minimum at 1 µm.
Figure 3 is an enlarged representation of the region of the graph shown in Figure 2 relating to particle dimensions between 0.5 and 2 µm. The graph reveals a pronounced local minimum of the response (ΔV₂₀) at about 1 µm, which represents the functional collapse point.
Figure 4 shows a flow chart of the process for the realization of the device, according to the invention.
Figure 5 shows a complete dataset acquired during a series of experimental tests of the medical device shown in Figure 1.

Further advantages and embodiments will result from the following detailed description.

### Detailed description

The device of the invention is defined as a passive device. Such characteristic is defined in particular by the micronized particles incorporated in the matrix and characterized by the specific relative dielectric constant between 2.0 and 5.0 measured at 1 MHz. Preferably, the incorporated micronized particles are employed for their polarization properties in response to endogenous electric fields generated by biological tissues. The device according to the invention is defined as passive in that it is not powered by any energy source, rather the action of the device is induced by an electric field, preferably a human bioelectric field. Advantageously, a characteristic molecular polarization is created in response to the human bioelectric field. Such molecular polarization generates a localized self-induced electric field that interacts permanently with the extracellular ionic microenvironment near the area of the skin where the device is applied, favoring the modulation of the local ionic distribution and the stabilization of the membrane potentials of the adjacent cells, without the need for external electrical power supply or active components.

As regards the micronized particles incorporated in the matrix, they are stably incorporated in the matrix. Preferably, the micronized particles incorporated in the matrix have dimensions greater than 1 µm and smaller than or equal to 20 µm. According to a preferred aspect, such particles present a coating based on functionalized silane agents.

Preferably, the micronized particles incorporated in the matrix have dimensions between 2 µm and 20 µm. According to a particularly preferred aspect of the invention, the linear dimension of said particles is between 5 µm and 10 µm. This interval corresponds to the region of maximum efficacy of the polarization response, guaranteeing a robust technical effect and significantly superior compared to particles of dimensions smaller or larger than such optimal window.

As regards the concentration of the micronized particles inside the matrix, preferably, the micronized particles are present in a weight fraction between 5% and 20% compared to the total weight of the composite material. As demonstrated by experimental validation, this interval represents an optimal functional-technological compromise to generate a sufficient density of internal interfaces for the maintenance of a polarization guaranteeing the producibility of the device.

As regards the silicone matrix, it is a cross-linkable matrix with relative dielectric constant between 2.8 and 3.5.

In a preferred embodiment, the relative dielectric constant of the silicone matrix is between 2.8 and 3.2. This narrower interval allows an optimal dielectric coupling between the matrix itself and the biological tissues, improving the distribution of the endogenous electric field and, consequently, the stability and intensity of the polarization response of the device.

As regards the passive functional layer, preferably it presents a thickness between 0.5 mm and 2.5 mm. Advantageously, such layer is flexible and biocompatible.

In some embodiments, the thickness of the passive functional layer is comprised in a thickness range between 2.5 mm and 4.0 mm, providing an optimal compromise between the stabilization dynamics and the robustness of the potential plateau generated.

The device according to the invention is advantageously effective thanks to its action on both excitable and non-excitable cells. Preferably the medical device of the invention acts on cells chosen from neurons, myocytes, glial cells, fibroblasts, endothelial cells and cells of the immune system.

According to a preferred aspect, the device further comprises a support layer configured to apply the device on a portion of skin. Preferably, the device of the invention is applied to the skin with a common bandage or adhesive support compatible with the epidermis.

By passive medical device, in the present text it is intended a device devoid of active components or external power supply. Such a device in fact exploits the dielectric and capacitive properties of the functional materials of which it is composed, generating a spontaneous polarization in response to the endogenous bioelectric fields of the tissues with which it comes into contact. Advantageously, this phenomenon has as results: a) a local redistribution of the ionic charges in the interface zone between device and tissue, b) the restoration of the homeostatic conditions of the extracellular fluid including also the areas of the excitable tissues (for example, neuronal membranes) and non-excitable ones, in therapeutic response for pathological states characterized by abnormal depolarization. The invention, therefore, allows obtaining a therapeutic effect analogous to that of microcurrents, without generating exogenous current or using electronic circuits, guaranteeing a non-invasive, continuous, safe treatment and applicable in clinical or home contexts.

Without wishing to be bound by any theory, the principle of operation, therefore, is based on the interaction with the endogenous bioelectric fields of the biological tissues, exploiting the intrinsic dielectric and capacitive properties of the materials of construction.

As regards the process of the present invention, it is a process for the realization of a flexible functional composite, obtained through the dispersion of micronized particles of a multilayer polymeric film based on polyester materials inside a cross-linked platinum silicone matrix.

As regards the process for the realization of the medical device according to the invention, such process, before step d), may include a further step c') of surface treatment of the particles by means of plasma or silane coating.

As known to the person skilled in the art, the cross-linking that occurs in step d) can be carried out, for example, thermally or at low temperature, depending on the nature of the catalytic system adopted, using for example open or closed molds, in static conditions or by means of injection molding processes. The medical device according to the invention is intended for cutaneous application on specific body regions in order to favor, through a mechanism of passive electrostatic interaction with the extracellular ionic environment, the modulation of the neuromuscular tone and the restoration of the electrochemical local equilibrium.

Such effects result useful in a variety of functional conditions, chosen from:
- Alterations of muscle tone, including states of hypertonia, spasticity or hypotonia;
- Disorders of proprioception and postural control;
- Rigidity and muscular contractures of neurogenic or mechanical origin;
- Alterations of peripheral nerve transmission in the presence of neuropathies or inflammations;
- Chronic pain associated with neuro-musculo-fascial dysfunctions;
- Neurodegenerative conditions wherein the membrane potential of excitable cells is altered (e.g. neurons, glia, myocytes);
- Support for post-traumatic or post-surgical neuromotor rehabilitation; and/or
- Regulation of the activity of the autonomic nervous system, with potential effects on deep relaxation and functional recovery.

The device may furthermore find use in the context of integrated medicine and physical therapies, in combination with or as an alternative to treatments such as microcurrent, biofeedback, or low-intensity somatosensory stimulation, without the need for external electrical energy or programming parameters

### Experimental validation of the dielectric response

To characterize the electrical response of the passive medical device for cutaneous application in contact with human skin, a series of experimental laboratory tests was conducted.

The medical device analyzed for the purposes of the experimental validation comprised a heterogeneous composite architecture, including a cross-linkable silicone matrix with a controlled relative dielectric constant (εᵣ = 2.7) and micronized dielectric polyester particles dispersed at definite weight fractions (φ) with a relative dielectric constant between 2.0 and 5.0 measured at 1 MHz. The composite was realized in layers with a controlled geometric thickness (t).

Measurements were performed on human subjects in static open-circuit conditions, using a high-impedance electrometer (Keithley 6512). The open-circuit potential (OCP) was measured at time intervals of 5, 10 and 20 minutes after application on the skin to observe the temporal evolution of the dielectric polarization.

The experiments were designed to independently evaluate the physical response of the device comprising the composite material consisting of the cross-linked silicone matrix and comprising a stable dispersion of micronized particles of polyester polymer material incorporated in the matrix, and to identify the influence of key microstructural parameters, including the particle dimension (d), the weight fraction of the particles (φ) and the thickness of the composite (t).

The experimental results demonstrate a non-monotonic dependence of the dielectric response dependent on the particle dimension (d). The data also reveal a distinct dimensional threshold for the activation of the interfacial polarization mechanism. In particular:
- For particle dimensions of about 1 µm, the measured potential difference (ΔV₂₀) collapses to values below 1 mV, exhibiting a local minimum around 1 µm indicating that the interfacial polarization mechanism is not effectively activated. In other words, the process of charge accumulation at the interface between the two materials does not occur or occurs in such a weak way as to be irrelevant.
- For particle dimensions equal to or greater than 2 µm, the potential difference increases by more than an order of magnitude, reaching a stable and robust plateau for micronized particles with dimensions between 1 µm and 20 µm. The optimal performance was observed in the interval 5-10 µm.
- Exploratory tests conducted with particles of larger dimensions (for example, 30-50 µm) showed a second collapse of the polarization response, with values of ΔV₂₀ reduced to about 1.5-3.7 mV.

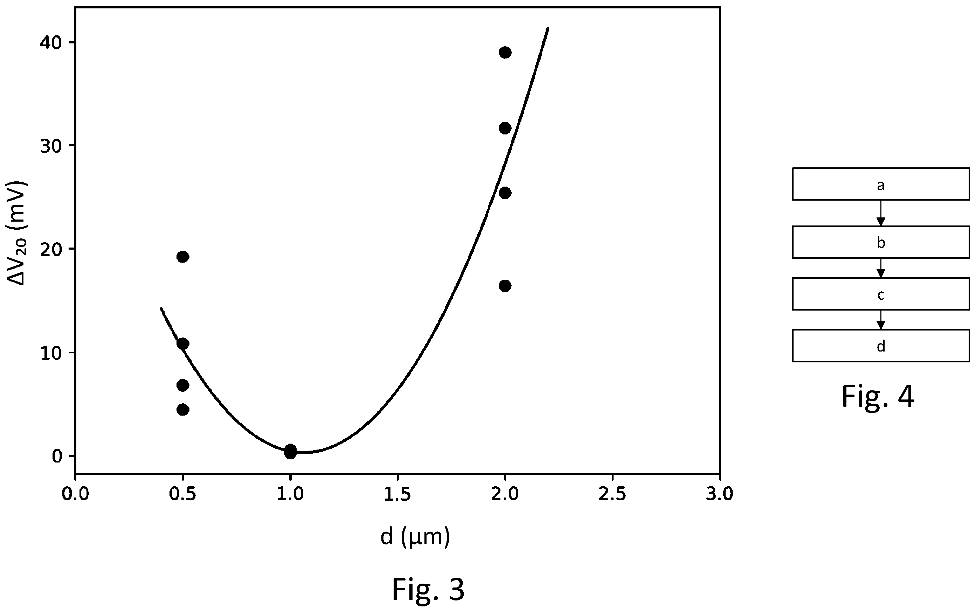

This behavior is illustrated in Figure 2 and in Figure 3 which demonstrates a non-linear generation of an electric potential difference on the surface of the device, which manifests when this is applied passively on the skin. Such electric potential difference results optimal within the functional window. In particular, the experimental investigation has identified a multi-parametric functional window that involves a measurable increase in the dielectric response.

Such multi-parametric window is characterized by:
- A particle dimension (d) ≥ 2 µm, preferably in the range 5-10 µm.
- A weight fraction of the particles (φ) of about 5-20% w/w, which represents a functional-technological compromise between dielectric performance and producibility (e.g. mixing viscosity, dispersion quality).
- A thickness of the composite layer (t), typically 2.5-4.0 mm, which acts as an active functional parameter that influences the stabilization dynamics and the robustness of the plateau.
- A relative dielectric constant of the matrix (εᵣ) in the range of about 2.5-3.2.

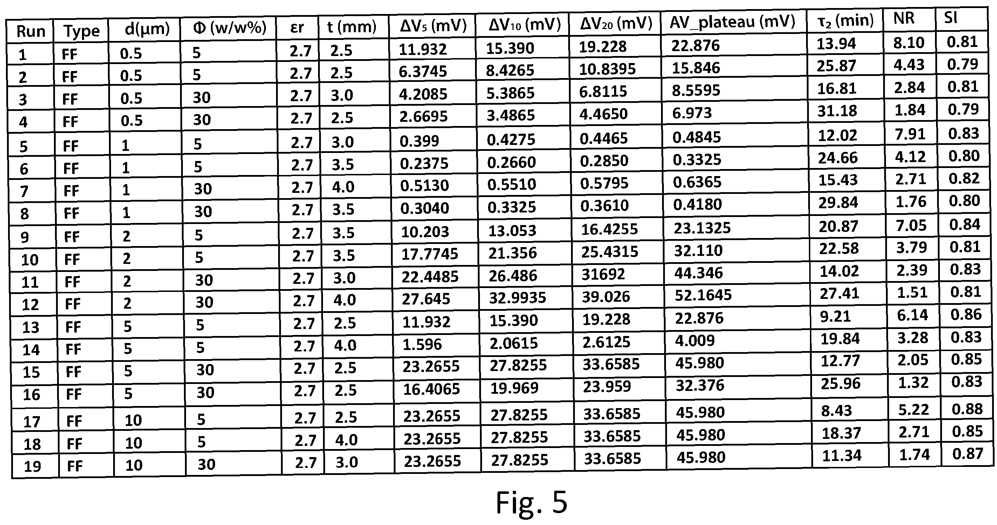

The table in Figure 5 presents some representative data illustrating the influence of the particle dimension on the potential difference measured after 20 minutes (ΔV₂₀) and on the steady-state plateau voltage (AV_plateau).

The parameters indicated in the table in Figure 5 are defined as follows: d (µm) is the diameter of the micronized polyester particles dispersed in the matrix; 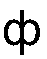 (w/w %) indicates the concentration of the polyester particles relative to the total weight of the composite; εᵣ is the intrinsic electrical property of the silicone matrix; t (mm) is the geometric thickness of the layer of the device tested; ΔV₅ (mV), ΔV₁₀ (mV) and ΔV₂₀ (mV) indicate the potential difference measured respectively after 5, 10 and 20 minutes from the application of the device on the skin, AV_plateau (mV) is the steady-state plateau voltage and represents the stable voltage value that the device reaches after the initial growth phase; τ₂ (min) indicates the slow time constant and indicates the speed with which the device reaches its plateau voltage; NR indicates the normalized response to compare the relative performances between different configurations; and SI represents the stability index which indicates a measure of how stable and free of fluctuations the voltage is once the plateau has been reached. A high SI value (for example, in the interval between 0.79 and 0.88) suggests a robust and reproducible behavior, without significant drifts over time.

As shown in the table in Figure 5, the response collapses drastically at d=1 µm (Trial 5) compared to the values at d=0.5 µm (Trial 1) and d ≥ 2 µm (Trials 12, 15, 17), confirming the existence of a multi-parametric functional window.

### Bibliography

1. Odell R.H. Jr., Sorgnard R.E. (2008). Anti-inflammatory Effects of Electronic Signal Treatment. Pain Physician Journal, 11:891-907.
2. Kloth L.C. (2014). Electrical Stimulation Technologies for Wound Healing. Advances in Wound Care, 3(2):81-90.
3. Hyytiäinen H.K. et al. (2023). A Systematic Review of Complementary and Alternative Veterinary Medicine: Electrotherapy. Animals, 13(64).
4. Lewis P.M. et al. (2016). Brain Neuromodulation Techniques: A Review. The Neuroscientist, 22(4):406-421.
5. Humphrey J.D., Dufresne E.R., Schwartz M.A. (2014). Mechanotransduction and extracellular matrix homeostasis. Nature Reviews Molecular Cell Biology, 15(12):802-812.
6. Di C., Yao Y., Wang Y., Zhang M. (2023). The Cellular and Molecular Basis of Mechanotransduction in the Extracellular Matrix and Cell Membrane Interface. Frontiers in Bioengineering and Biotechnology, 11:1182160.
7. Frantz C., Stewart K.M., Weaver V.M. (2010). The Extracellular Matrix at a Glance. Journal of Cell Science, 123(24):4195-4200

## Claims

1. Medical device for cutaneous application comprising a composite material made of a cross-linked silicone matrix comprising a stable dispersion of micronized particles of polyester polymer material incorporated in said matrix, said particles having a relative dielectric constant between 2.0 and 5.0 measured at 1 MHz.

2. Medical device for cutaneous application according to claim 1, wherein said composite material constitutes a single functional layer, in which the micronized particles are homogeneously dispersed within the cross-linked silicone matrix, defining a plurality of internal dielectric interfaces.

3. Medical device according to any one of the preceding claims, further comprising a support layer configured to apply the device on a portion of skin.

4. Medical device according to any one of the preceding claims, wherein the micronized particles have dimensions greater than 1 µm and less than or equal to 20 µm, and are stably incorporated in the matrix, preferably wherein the micronized particles have dimensions between 5 µm and 10 µm.

5. Medical device according to any one of the preceding claims, wherein said particles have a coating based on functionalized silane agents.

6. Medical device according to any one of the preceding claims, wherein said silicone matrix is a cross-linkable elastomeric matrix with a relative dielectric constant between 2.8 and 3.5 and preferably wherein the relative dielectric constant of said silicone matrix is between 2.8 and 3.2.

7. Medical device according to any one of the preceding claims, wherein the thickness of the composite layer is between 0.5 mm and 2.5 mm, and it is flexible and biocompatible.

8. Medical device according to any one of claims 1-6, wherein the thickness of the composite layer is between 2.5 mm and 4.0 mm.

9. Medical device according to any one of the preceding claims, wherein said micronized particles are present in a weight fraction between 5% and 20% relative to the total weight of the composite material.

10. Process for the manufacture of a medical device according to any one of the preceding claims, comprising the steps of:
a) fragmentation by mechanical cutting of a layer or multilayer of polyester polymer material having a relative dielectric constant between 2.0 and 5.0 measured at 1 MHz to obtain fragments of the layer or multilayer of polymer material;
b) cryogenic micronization by grinding the fragments referred to in step a) in liquid nitrogen, under conditions below the glass transition point of the polyester polymer material, to obtain micronized particles of linear dimension greater than 1 µm and less than or equal to 20 µm;
c) dispersion of the micronized particles referred to in point b) in a pre-crosslinkable silicone composition containing vinyl groups, under inert conditions and controlled stirring;
d) addition to the composition of a cross-linking agent suitable to trigger the formation of a cross-linked silicone matrix, inside which the micronized particles are permanently incorporated.
